(19) Europäisches Patentamt
European Patent Office
Office européen des brevets

(11) Numéro de publication: **0 085 265 B1**

(12) **FASCICULE DE BREVET EUROPEEN**

(45) Date de publication du fascicule du brevet: **22.05.85**

(51) Int. Cl.⁴: **C 07 C 45/46**, C 07 C 49/84, C 07 C 79/36, C 07 C 149/34

(21) Numéro de dépôt: **82401214.0**

(22) Date de dépôt: **30.06.82**

(54) **Procédé de préparation de alpha,alpha-difluoroalkoxy ou alpha,alpha-difluoroalkylthiophénylcétones.**

(30) Priorité: **21.01.82 FR 8200876**

(43) Date de publication de la demande: **10.08.83 Bulletin 83/32**

(45) Mention de la délivrance du brevet: **22.05.85 Bulletin 85/21**

(84) Etats contractants désignés: **AT BE CH DE FR GB IT LI LU NL SE**

(56) Documents cités:
**EP - A - 0 069 598**
**GB - A - 2 030 158**
**GB - A - 2 045 760**
**US - A - 3 732 307**
**US - A - 4 276 226**

(73) Titulaire: **RHONE-POULENC SPECIALITES CHIMIQUES, "Les Miroirs" 18, Avenue d'Alsace, F-92400 Courbevoie (FR)**

(72) Inventeur: **Desbois, Michel, 526, chemin du Bois, F-69140 Rillieux (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

**EP 0 085 265 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation d'α,α-difluoroalkoxy- ou α,α-difluoroalkylthiophénylcétones.

On connaît dans l'art antérieur des documents décrivant la préparation de composés de ce type.

On peut citer, notamment, la demande de brevet français publiée sous le numéro 2272079, qui décrit en particulier la préparation de l'isopropyl-p-trifluorométhoxyphénylcétone, selon laquelle on prépare un réactif de Grignard à partir d'oxyde de p-bromophényle et de trifluorométhyle et de magnésium. On le fait réagir avec de l'isobutyronitrile à reflux. Le produit est hydrolysé pour donner la cétone recherchée. On peut encore citer la demande de brevet français publiée sous le numéro 2194422 qui décrit un procédé analogue.

Ce type de procédé présente de sérieux inconvénients qui ne le rendent pas attractif au plan industriel. En effet, les étapes sont nombreuses et les temps de réaction sont longs. De plus, les rendements sont médiocres puisqu'ils n'atteignent que 30 à 40%. D'autre part, et cela n'est sûrement pas le moins important, la mise en œuvre d'un organomagnésium et des solvants qui l'accompagnent est dangereuse pour l'environnement: il est nécessaire de prendre des précautions au plan technique, ce qui alourdit l'économie du procédé.

On connaît encore dans l'art antérieur des documents (voir par exemple Olah, «Friedel-Crafts and Related Reactions», III, Part I, 1964, Interscience Publishers, p. 8 ss.) qui décrivent l'acylation de substrats aromatiques (autres que les trifluorométhoxybenzènes ou trifluorométhylthiobenzènes) en présence de catalyseurs de Friedel et Crafts comme $AlCl_3$.

Les expériences de la titulaire ont montré que les catalyseurs de Friedel et Crafts comme $AlCl_3$ sont inefficaces lorsque le composé aromatique de départ comporte un groupement $OCF_3$ ou $SCF_3$. Ces derniers sont même dégradés.

En présence d'acide sulfurique, autre catalyseur classique, les groupements $OCF_3$ et $SCF_3$ sont de même dégradés.

Il est également connu dans l'art antérieur des brevets US N° 4276226 et GB N° 203058 décrivant la condensation de dérivés phénoliques avec des acides ou des diacides en milieu acide fluorhydrique et en présence de trifluorure de bore. Les substrats aromatiques sur lesquels sont effectuées les acylations sont tous des substrats activés par la présence du groupe hydroxy, sur lesquels les acylations peuvent être effectuées en présence soit de $BF_3$ seul, soit d'acide fluorhydrique seul. Les substrats du présent brevet, polyhalogénoalkoxybenzènes et polyhalogénoalkylthiobenzènes, sont des dérivés désactivés sur lesquels l'acylation n'est pas possible en présence de $BF_3$ seul ou d'acide fluorhydrique seul.

Il est encore connu, d'après la demande de brevet européen N° 69598, un procédé de préparation de diphénylcétones par condensation de dérivés benzéniques halogénés avec des composés acylants en présence d'un catalyseur comprenant un mélange d'acide de Lewis et d'acide fort. L'acide de Lewis peut être $BF_3$ et l'acide fort l'acide fluorhydrique.

La titulaire a maintenant découvert les conditions opératoires permettant de mettre en œuvre la réaction d'acylation sur les substrats aromatiques ayant un substituant polyhalogénoalkoxy, notamment trihalogénométhoxy ou polyhalogénoalkylthio et, notamment, trihalogenométhylthio, ce qui n'avait pu être obtenu dans l'art antérieur.

La présente invention concerne un procédé de préparation de phénylcétones par réaction d'un dérivé benzénique avec un acide carboxylique, un précurseur ou un dérivé de cet acide, en présence de trifluorure de bore en quantité telle que la pression absolue de trifluorure de bore dans l'enceinte réactionnelle soit supérieure à 1 bar et en présence d'acide fluorhydrique comme solvant, procédé caractérisé en ce que le dérivé benzénique est désactivé par au moins un groupe α,α-difluoroalkoxylé ou par au moins un groupe α,α-difluorothioalkyle.

On connaît dans l'art antérieur une publication de MM. Buu-Hoi et Xuong dans «Journal of Organic Chemistry», vol. 26, juillet 1961, pp. 2401-2402, qui décrit la synthèse de cétones phénoliques dérivées de di- et triphénols et d'α- et β-naphtol par condensation d'acides carboxyliques avec les phénols en présence du gaz produit par la réaction de l'oléum sur le fluoroborate de potassium. Ce gaz contient du trifluorure de bore et de l'acide fluorhydrique, à côté d'autres composés comme l'acide fluorosulfonique principalement. Selon cet article, l'acide fluorhydrique exhalte les propriétés condensantes du trifluorure de bore.

Il est à noter que la réaction décrite dans cette publication a lieu en milieu solvant comme le xylène. Il est à noter de plus que le composé aromatique envisagé est un phénol, donc un benzène comportant un substituant activant (OH). Un tel procédé n'est pas transposable à la préparation d'α,α-difluoroalkoxy- ou α,α-difluoroalkylthiophénylcétones dans la mesure où le solvant réagirait avec l'acide carboxylique, son précurseur ou son dérivé, le polyhalogénoalkoxy- ou polyhalogénoalkylthiobenzène étant moins activé que le xylène.

D'autre part, le gaz issu de l'attaque par l'oléum du fluoroborate de potassium ne peut être assimilé au couple trifluorure de bore/acide fluorhydrique mis en œuvre dans le procédé selon l'invention. En effet, dans ce dernier, l'acide fluorhydrique à l'état liquide joue le rôle de solvant, alors que dans le document cité il est sous forme gazeuse.

Au sens de la présente invention, on entend par polyhalogénoalkoxybenzène ou polyhalogénoalkylthiobenzène, d'une part, ces produits proprement dits et, d'autre part, leurs homologues correspondant à la présence d'un ou de plusieurs radicaux substituant le noyau benzénique.

Plus particulièrement, les polyhalogénoalkoxybenzènes ou polyhalogénoalkylthiobenzènes concernés par la présente invention ont pour formule générale:

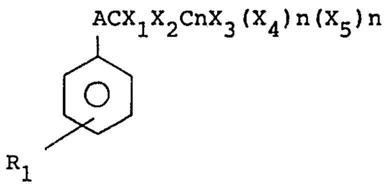

(I)

dans laquelle:

$X_1$ et $X_2$, identiques ou différents, représentent Cl, Br, I ou F,

$X_3$, $X_4$ et $X_5$, identiques ou différents, représentent H, Cl, Br, I ou F,

n est un nombre entier supérieur ou égal à 0 et inférieur ou égal à 5 ($0 \leqslant n \leqslant 5$),

A représente O ou S, et

$R_1$ représente au moins un élément choisi parmi le groupe comprenant l'hydrogène et les radicaux alkyle et alkoxy ayant de 1 à 6 atomes de carbone, les radicaux phényle et phénoxy substitués par au moins un groupement plus désactivant que le groupement $ACX_1X_2CnX_3(X_4)n(X_5)n$, OH, Cl, Br, I et F.

Les radicaux $R_1$ phényle et phénoxy doivent être substitués par des groupements plus désactivants que le groupement $ACX_1X_2CnX_3(X_4)n(X_5)n$ pour que la réaction d'acylation se fasse sur le noyau benzénique porteur du groupement $ACX_1X_2CnX_3(X_4)n(X_5)n$. Dans le cas contraire, la réaction d'acylation se ferait sur le radical phényle ou phénoxy. On peut citer comme exemples de tels groupements plus désactivants les groupements COOH, CN, $NO_2$, $CX_1X_2X_3$ et cétones.

Les composés de formule I dans laquelle n = 0 ou 1 et $X_1$, $X_2$ et $X_3$ sont identiques sont encore plus particulièrement concernés par la présente invention. On préfère mettre en œuvre parmi ceux-ci les composés dans lesquels $X_1$, $X_2$ et $X_3$ représentent le fluor.

On peut citer comme exemples de composés de formule I les composés suivants: le trifluorométhoxybenzène, le trifluorométhylthiobenzène, l'o-, m- et p-chlorotrifluorométhoxybenzène, l'o-, m- et p-chlorotrifluorométhylthiobenzène, l'o-, m- et p-bromotrifluorométhylthiobenzène, l'o-, m- et p-bromotrifluorométhoxybenzène, l'o-, m- et p-méthyltrifluorométhoxybenzène, l'o-, m- et p-méthyltrifluorométhylthiobenzène, l'o-, m- et p-méthoxytrifluorométhoxybenzène, l'o-, m- et p-méthoxytrifluorométhylthiobenzène, l'o-, m- et p-hydroxytrifluorométhoxybenzène, l'o-, m- et p-hydroxytrifluorométhylthiobenzène, le trifluorométhyl-4-trifluorométhoxy-4'-biphényle, le nitro-3-trifluorométhoxy-4'-diphényloxyde (on peut également citer les homologues chlorés, bromés ou iodés des composés ci-dessus), le difluorobromométhoxybenzène, le difluorobromométhylthiobenzène, le dichlorofluorométhoxybenzène, le dichlorofluorométhylthiobenzène, le difluorochlorométhoxybenzène, le difluorochlorométhylthiobenzène, l'α,α,β,β,β-pentachloroéthoxybenzène, l'α,α,β,β,β-pentachloroéthylthiobenzène; le difluorométhoxybenzène; le difluorométhylthiobenzène; l'α,α,β,β-tétrafluoroéthoxybenzène; l'α,α,β,β-tétrafluoroéthylthiobenzène; l'α,α,β,β-tétrafluoro-β-bromoéthoxybenzène; l'α,α,β,β-tétrafluoro-β-bromoéthylthiobenzène; l'α,α-difluoro-β,β,β-trichloroéthoxybenzène.

Au sens de la présente invention, on entend par acide carboxylique le précurseur ou le dérivé de cet acide, tous les réactifs d'acylation bien connus dans l'art antérieur.

Selon un mode de réalisation particulier de la présente invention, l'acide carboxylique, le précurseur ou le dérivé de cet acide répond à la formule générale:

$$R_2-COX_6 \qquad (II)$$

dans laquelle:

$R_2$ représente un radical aliphatique ou aromatique,

$X_6$ représente un halogène, un groupe dérivant de l'anion d'un acide inorganique, OH, $OR_3$, $OCOR_4$, $NH_2$, $NHR_5$ ou $NR_6R_7$, où $R_3$, $R_4$, $R_5$, $R_6$ et $R_7$ sont un radical aromatique ou aliphatique.

On peut citer comme exemples de groupes dérivant de l'anion d'un acide inorganique $ClO_4^-$ et $BF_4^-$.

L'invention est particulièrement bien adaptée à la mise en œuvre d'un composé de formule II dans laquelle $R_2$ représente un radical alkyle, phényle, alkylphényle, phénylalkyle ou phényle comportant au moins un substituant, par exemple un halogène, $NO_2$, CN, $NH_2$, COOH.

On peut citer comme exemples de tels composés le chlorure d'acétyle, l'acide acétique, l'anhydride acétique, le chlorure de benzoyle, l'acide benzoïque, l'anhydride benzoïque, le chlorure d'orthochlorobenzoyle, le chlorure de parachlorobenzoyle, le chlorure de parafluorobenzoyle, le fluorure de paratrifluorométhylbenzoyle, le fluorure d'orthotrifluorométhylbenzoyle, le chlorure de paranitrobenzoyle, l'acide paranitrobenzoïque, l'acide paraaminobenzoïque, le chlorure d'isobutyroyle, l'acide isobutyroïque, l'acide propanoïque, le chlorure de propanoyle, le chlorure de paratoluyle, le chlorure de parabenzylbenzoyle.

Le procédé selon l'invention est mis en œuvre de préférence en utilisant une quantité d'acide fluorhydrique telle que le rapport molaire de l'acide fluorhydrique au composé de formule I est compris entre 5 et 50. Encore plus préférentiellement, ce rapport est compris entre 10 et 30.

L'acide fluorhydrique mis en œuvre est de préférence anhydre. La mise en œuvre d'acide fluorhydrique aqueux entraînerait une consommation inutile de trifluorure de bore sous forme de HF, $BF_3$, $H_2O$ ($H_3O^+BF_4^-$).

Les composés de formules I et II sont utilisés en quantités sensiblement équimolaires. Un léger excès du premier peut cependant être souhaitable.

On préfère plus particulièrement utiliser une quantité de trifluorure de bore telle que la pression absolue de $BF_3$ dans l'enceinte réactionnelle soit comprise entre 6 et 20 bar. Plus la pression sera élevée, plus la vitesse de réaction augmentera. Une pression supérieure à 20 bar n'est pas exclue du domaine de l'invention, mais n'apporte pas d'avantage particulier. L'homme de l'art adaptera donc la pression à l'économie du procédé.

Le procédé selon l'invention est de préférence mis en œuvre à une température comprise entre −20 et 150° C.

Les temps de réaction sont généralement compris entre quelques minutes et plusieurs heures.

Les α,α-difluoroalkoxy- ou α,α-difluoroalkylthiophénylcétones obtenues selon le procédé selon l'invention ont pour formule générale:

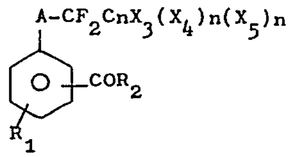

(III)

où A, $R_1$, $R_2$, $X_3$, $X_4$, $X_5$ et n ont la signification précédente.

Lors de la réaction qui s'effectue en milieu HF, quand dans le composé de formule I n = 0 et $X_3$ est un halogène, le composé obtenu comportera le substituant $A-CF_3$ ($A-CCl_3$, $A-CBr_3$, $A-CI_3$, $A-CF_2Br$, $A-CCl_2F$, $ACF_2Cl$, etc., se transforment en $A-CF_3$). Quand n = 0 et $X_3$ est H, le composé obtenu comportera le substituant $A-CF_2H$. Quand n est supérieur à 0, seuls les substituants $X_1$ et $X_2$ sont échangés par des atomes de fluor, sauf s'ils sont initialement des fluor.

La position du groupement $-COR_2$ par rapport aux groupements $A-CF_2CnX_3(X_4)n$ et $R_1$ obéit aux règles de substitution bien connues du chimiste organicien.

Les cétones obtenues par le procédé de l'invention sont utiles, notamment comme intermédiaires de synthèse de composés ayant une activité pharmaceutique ou phytosanitaire.

On peut citer comme exemples de composés pouvant être préparés par le procédé selon l'invention: trifluorométhoxy-4-chloro-2'-benzophénone, trifluorométhoxy-4-chloro-4'-benzophénone, trifluorométhoxy-4-benzophénone, trifluorométhoxy-4-acétophénone, trifluorométhoxy-4-isobutyrophénone, trifluorométhoxy-4-fluoro-4'-benzophénone, trifluorométhoxy-4-trifluorométhyl-4'-benzophénone, trifluorométhoxy-4-trifluorométhyl-2'-benzophénone, trifluorométhoxy-4-chloro-2-nitro-4'-benzophénone, trifluorométhoxy-2-méthyl-5-amino-4'-benzophénone, trifluorométhoxy-2-chloro-4-fluoro-4'-benzophénone, trifluorométhoxy-3-hydroxy-4-cyano-3'-benzophénone, trifluorométhoxy-3-dichloro-4',6-benzophénone, trifluorométhoxy-2-dichloro-4',5-benzophénone, difluorométhoxy-4-fluoro-4'-benzophénone, (α,α-difluoro-β,β,β-trichloroéthoxy)-4-chloro-4'-benzophénone, α,α,β,β-tétrafluoroéthoxy-4-fluoro-4'-benzophénone, (α,α,β,β-tétrafluoro-β-bromoéthoxy)-4-fluoro-2'-benzophénone; on peut également citer les homologues soufrés de ces composés.

L'invention va être maintenant plus complètement décrite à l'aide des exemples qui vont suivre. Ceux-ci ne sauraient être considérés comme limitant de façon quelconque l'invention.

*Exemple 1*

Dans un réacteur inox de 250 ml muni d'un système d'agitation magnétique, on introduit aux environs de 0° C 100 ml de HF anhydre, 23,6 g (0,3 mol) de chlorure d'acétyle et 32,4 g (0,2 mol) de trifluorométhoxybenzène. On ferme le réacteur, puis on introduit du trifluorure de bore ($BF_3$) gazeux jusqu'à la pression constante de 6 bar. On laisse alors réagir sous agitation pendant 3 h à la température ambiante. Après réaction, on décomprime le réacteur jusqu'à pression atmosphérique, puis on coule le mélange réactionnel sur 200 g de glace pilée. On extrait alors le mélange hétérogène ainsi obtenu par 3 fois 200 $cm^3$ de chlorure de méthylène. Les phases organiques sont lavées par 3 fois 200 $cm^3$ d'eau, 1 fois 200 $cm^3$ d'une solution aqueuse à 3% de potasse et 2 fois 200 $cm^3$ d'eau. La phase organique ainsi traitée est séchée sur sulfate de magnésium et le solvant est éliminé par distillation sous pression réduite. On recueille ainsi 37,2 g (rendement: 91%) de p-trifluorométhoxyacétophénone à 96,3% de pureté.

*Exemple 2*

On procède comme à l'exemple 1 avec les composés et conditions suivants:

| | |
|---|---|
| Acide fluorhydrique anhydre: | 100 g |
| Trifluorométhoxybenzène: | 32,4 g (0,2 mol) |
| Chlorure d'orthochlorobenzoyle: | 35 g (0,2 mol) |
| Trifluorure de bore: | 6 bar à 20° C |
| Température: | 20° C |
| Durée: | 1 h |

On recueille 53,3 g (rendement: 88,7%) de trifluorométhoxy-4-chloro-2'-benzophénone liquide, de pureté 95,5%.

*Exemple 3*

On procède comme à l'exemple 1 avec les composés et conditions suivants:

| | |
|---|---|
| Acide fluorhydrique anhydre: | 100 g |
| Trifluorométhoxybenzène: | 32,4 g (0,2 mol) |
| Chlorure d'isobutyroyle: | 23 g (0,216 mol) |
| Trifluorure de bore: | 10 bar à 20° C |
| Température: | 20° C |
| Durée: | 20 h |

On recueille 27,8 g (rendement: 60%) de p-trifluorométhoxyisobutyrophénone liquide, de pureté 70%. Une simple distillation permet de recueillir 17 g de p-trifluorométhoxyisobutyrophénone, de pureté 99,5% (Eb.: 105° C sous 10 mmHg).

*Exemple 4*

On procède comme à l'exemple 1 avec les composés et conditions suivants:

| | |
|---|---|
| Acide fluorhydrique anhydre: | 100 g |
| Trifluorométhoxybenzène: | 32,4 g (0,2 mol) |
| Chlorure de p-chlorobenzoyle: | 35 g (0,2 mol) |
| Trifluorure de bore: | 10 bar à 20° C |
| Température: | 5° C |
| Durée: | 3 h |

On recueille 55,1 g (rendement: 91,7%) de trifluorométhoxy-4-chloro-4'-benzophénone à 98,2% de pureté, p. f. 74,5-75° C (MeOH).

*Exemple 5*

On procède comme à l'exemple 1 avec les composés et conditions suivants:

| | |
|---|---|
| Acide fluorhydrique anhydre: | 100 g |
| Trifluorométhoxybenzène: | 32,4 g (0,2 mol) |
| Chlorure de p-fluorobenzoyle: | 31,7 g (0,2 mol) |
| Trifluorure de bore: | 8 bar à 20° C |
| Température: | 20° C |
| Durée: | 24 h |

On recueille 52,2 g (rendement: 92%) de trifluorométhoxy-4-fluoro-4'-benzophénone à 99% de pureté, p. f. 42-43° C (MeOH).

*Exemple 6*

On procède comme à l'exemple 1 avec les composés et conditions suivants:

| | |
|---|---|
| Acide fluorhydrique anhydre: | 100 g |
| Trifluorométhoxybenzène: | 32,4 g (0,2 mol) |
| Anhydride benzoïque: | 22,6 g (0,1 mol) |
| Trifluorure de bore: | 10 bar à 20° C |
| Température: | 40° C |
| Durée: | 2 h |

On recueille 45,8 g (rendement: 86%) de trifluorométhoxy-4-benzophénone à 95% de pureté.

*Exemple 7*

On procède comme à l'exemple 1 avec les composés et conditions suivants:

| | |
|---|---|
| Acide fluorhydrique anhydre: | 100 g |
| Trifluorométhoxybenzène: | 32,4 g (0,2 mol) |
| Acide benzoïque: | 24,4 g (0,2 mol) |
| Trifluorure de bore: | 10 bar à 20° C |
| Température: | 50° C |
| Durée: | 3 h |

On recueille 40,3 g (rendement: 75,7%) de trifluorométhoxy-4-benzophénone à 96% de pureté.

*Exemple 8*

On procède comme à l'exemple 1 avec les composés et conditions suivants:

| | |
|---|---|
| Acide fluorhydrique anhydre: | 100 g |
| Trifluorométhoxybenzène: | 32,4 g (0,2 mol) |
| Fluorure de p-trifluorométhylbenzoyle: | 38,4 g (0,2 mol) |
| Trifluorure de bore: | 6 bar à 20° C |
| Température: | 30° C |
| Durée: | 1 h |

On recueille 48 g (rendement: 71,8%) de trifluorométhyl-4-trifluorométhoxy-4'-benzophénone à 99,2% de pureté, p. f. 61,5-62° C (MeOH).

*Exemple 9*

On procède comme à l'exemple 1 avec les composés et conditions suivants:

| | |
|---|---|
| Acide fluorhydrique anhydre: | 100 g |
| Trifluorométhoxybenzène: | 32,4 g (0,2 mol) |
| Fluorure d'o-trifluorométhylbenzoyle: | 38,4 g (0,2 mol) |
| Trifluorure de bore: | 6 bar à 20° C |
| Température: | 30° C |
| Durée: | 2 h |

On recueille 61 g (rendement: 91,3%) de trifluorométhyl-2-trifluorométhoxy-4'-benzophénone liquide à 95% de pureté.

*Exemple 10*

On procède comme à l'exemple 1 avec les composés et conditions suivants:

| | |
|---|---|
| Acide fluorhydrique anhydre: | 130 g |
| Trifluorométhoxybenzène: | 32,4 g (0,2 mol) |
| Chlorure d'isobutyroyle: | 23,5 g (0,22 mol) |
| Trifluorure de bore: | 23 bar à 0° C |
| Température: | −10° C |
| Durée: | 18 h |

On recueille 38,5 g (rendement: 90%) de p-trifluorométhoxyisobutyrophénone à 96% de pureté.

*Exemple 11*

On procède comme à l'exemple 1 avec les composés et conditions suivants:

| | |
|---|---|
| Acide fluorhydrique anhydre: | 300 g |
| Trifluorométhoxybenzène: | 97,2 g (0,6 mol) |
| Chlorure d'isobutyroyle: | 64 g (0,6 mol) |
| Trifluorure de bore: | 15 bar à −15° C |
| Température: | −15° C |
| Durée: | 18 h |

On recueille 102,6 g (rendement: 80%) de p-trifluorométhoxyisobutyrophénone à 95% de pureté.

Dans cet exemple, on a procédé à l'élimination du HF solvant par une distillation sous pression réduite (100 mmHg) à 15° C.

*Exemple 12*

On procède comme à l'exemple 1 avec les composés et conditions suivants:

| | |
|---|---|
| Acide fluorhydrique anhydre: | 100 g |
| Trifluorométhylthiobenzène: | 17,8 g (0,1 mol) |
| Chlorure de p-fluorobenzoyle: | 15,8 g (0,1 mol) |
| Trifluorure de bore: | 10 bar à 20° C |
| Température: | 20° C |
| Durée: | 4 h |

On recueille 27 g (rendement: 92%) de trifluorométhylthio-4-fluoro-4'-benzophénone à 93% de pureté.

*Exemple 13*

On procède comme à l'exemple 1 avec les composés et conditions suivants:

| | |
|---|---|
| Acide fluorhydrique anhydre: | 100 g |
| Trichlorométhoxybenzène: | 42,3 g (0,2 mol) |
| Chlorure de p-fluorobenzoyle: | 31,7 g (0,2 mol) |
| Trifluorure de bore: | 10 bar à 20° C |
| Température: | 50° C |
| Durée: | 2 h |

On recueille 50,2 g (rendement: 88,5%) de trifluorométhoxy-4-fluoro-4'-benzophénone à 94% de pureté.

On note au cours de la réaction une forte augmentation de pression due au départ d'acide chlorhydrique provenant de l'échange Cl-F.

*Exemple 14*

On procède comme à l'exemple 1 avec les composés et conditions suivants:

| | |
|---|---|
| Acide fluorhydrique anhydre: | 100 g |
| Difluorobromométhoxybenzène: | 44,6 g (0,2 mol) |

Chlorure de p-chlorobenzoyle: 35 g (0,2 mol)
Trifluorure de bore: 10 bar à 20° C
Température: 20° C
Durée: 3 h

On recueille 53 g (rendement: 85%) de trifluorométhoxy-4-chloro-4'-benzophénone à 94% de pureté.

On note au cours de la réaction une augmentation de pression due au départ d'acide bromhydrique provenant de l'échange Br-F.

*Exemple 15*

On procède comme à l'exemple 1 avec les composés et conditions suivants:

Acide fluorhydrique anhydre: 100 g
Trichlorométhylthiobenzène: 22,7 g (0,1 mol)
Chlorure de p-fluorobenzoyle: 15,8 g (0,1 mol)
Trifluorure de bore: 15 bar à 20° C
Température: 50° C
Durée: 8 h

On recueille 25 g (rendement: 85%) de trifluorométhylthio-4-fluoro-4-benzophénone à 91% de pureté.

On note au cours de la réaction une augmentation de pression due au départ d'acide chlorhydrique provenant de l'échange Cl-F.

*Exemple 16*

On procède comme à l'exemple 1 avec les composés et conditions suivants:

Acide fluorhydrique anhydre: 100 g
p-Chlorotrifluorométhoxybenzène: 19,7 g (0,1 mol)
Chlorure de p-chlorobenzoyle: 17,5 g (0,1 mol)
Trifluorure de bore: 6 bar à 20° C
Température: 120° C
Durée: 18 h

On recueille 8,7 g (rendement: 26%) d'un mélange brut de trifluorométhoxy-3-dichloro-4',6-benzophénone et de trifluorométhoxy-2-dichloro-4',5-benzophénone.

*Exemple 17*

On procède comme à l'exemple 1 avec les composés et conditions suivants:

Acide fluorhydrique anhydre: 100 g
α,α-Difluorométhoxybenzène: 28,8 g (0,2 mol)
Chlorure de p-fluorobenzoyle: 31,7 g (0,2 mol)
Trifluorure de bore: 10 bar à 20° C
Température: 30° C
Durée: 3 h

On recueille 45,5 g (rendement: 85%) de difluorométhoxy-4-fluoro-4'-benzophénone brute.

*Exemple 18*

On procède comme à l'exemple 1 avec les composés et conditions suivants:

Acide fluorhydrique anhydre: 50 g
α,α,β,β,β-Pentachloroéthoxybenzène: 5 g (0,017 mol)
Chlorure de p-chlorobenzoyle: 3 g (0,017 mol)
Trifluorure de bore: 6 bar à 20° C
Température: 20° C
Durée: 4 h

On recueille 6 g (rendement: 88%) d'(α,α-difluoro,β,β,β-trichloroéthoxy)-4-chloro-4'-benzophénone brute.

*Exemple 19*

On procède comme à l'exemple 1 avec les composés et conditions suivants:

Acide fluorhydrique anhydre: 20 g
α,α,β,β-Tétrafluoroéthylthiobenzène: 1 g (0,005 mol)
Chlorure de p-fluorobenzoyle: 0,7 g (0,005 mol)
Trifluorure de bore: 12 bar à 20° C
Température: 0° C
Durée: 3 h

On recueille 1,2 g (rendement: 72%) d'(α,α,β,β-tétrafluoroéthylthio)-4-fluoro-4'-benzophénone brute.

*Exemple 20*

On procède comme à l'exemple 1 avec les composés et conditions suivants:

Acide fluorhydrique anhydre: 30 g
α,α,β,β-Tétrafluoro-β-bromoéthylthiobenzène: 2 g (0,007 mol)
Chlorure d'o-fluorobenzoyle: 1,5 g (0,009 mol)
Trifluorure de bore: 12 bar à 20° C
Température: 0° C
Durée: 4 h

On recueille 2,1 g (rendement: 75%) d'(α,α,β,β-tétrafluoro,β-bromoéthylthio)-4-fluoro-2'-benzophénone brute.

## Revendications

1. Procédé de préparation de phénylcétones par réaction d'un dérivé benzénique avec un acide carboxylique, un précurseur ou un dérivé de cet acide, en présence de trifluorure de bore en quantité telle que la pression absolue de trifluorure de bore dans l'enceinte réactionnelle soit supérieure à 1 bar et en présence d'acide fluorhydrique comme solvant, caractérisé en ce que le dérivé benzénique est désactivé par au moins un groupe α,α-difluoroalkoxylé ou par au moins un groupe α,α-difluorothioalkyle.

2. Procédé selon la revendication 1, caractérisé en ce que le dérivé benzénique désactivé a pour formule:

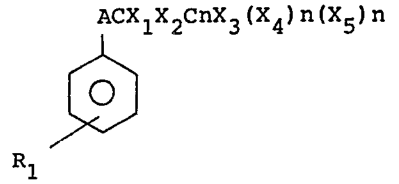

(I)

dans laquelle:

$X_1$ et $X_2$, identiques ou différents, représentent Cl, Br, I ou F,

$X_3$, $X_4$ et $X_5$, identiques ou différents, représentent H, Cl, Br, I ou F,

n est un nombre entier supérieur ou égal à 0 et inférieur ou égal à 5,

A représente O ou S, et

$R_1$ représente au moins un élément choisi parmi le groupe comprenant l'hydrogène et les radicaux alkyle et alkoxy ayant de 1 à 6 atomes de carbone environ, les radicaux phényle et phénoxy substitués par au moins un groupement plus désactivant que le groupement $ACX_1X_2CnX_3(X_4)n(X_5)n$, OH, Cl, Br, I et F.

3. Procédé selon la revendication 2, caractérisé en ce que dans la formule (I) n = 0 ou 1 et $X_1$, $X_2$ et $X_3$ sont identiques.

4. Procédé selon la revendication 3, caractérisé en ce que $X_1$, $X_2$ et $X_3$ représentent le fluor.

5. Procédé selon l'une des revendications principales, caractérisé en ce que l'acide carboxylique, le précurseur ou le dérivé de cet acide répond à la formule:

$$R_2-COX_6 \qquad (II)$$

dans laquelle:

$R_2$ représente un radical aliphatique ou aromatique,

$X_6$ représente un halogène, un groupe dérivant de l'anion d'un acide inorganique, OH, $OR_3$, $OCOR_4$, $NH_2$, $NHR_5$ ou $NR_6R_7$, où $R_3$, $R_4$, $R_5$, $R_6$ et $R_7$ sont un radical aromatique ou aliphatique.

6. Procédé selon la revendication 5, caractérisé en ce que dans la formule (II) $R_2$ représente un radical alkyle, phényle, alkylphényle, phénylalkyle ou phényle comportant au moins un substituant halogène, $NO_2$, CN, $NH_2$, COOH.

7. Procédé selon l'une des revendications précédentes, caractérisé en ce que on utilise une quantité d'acide fluorhydrique telle que le rapport molaire de l'acide fluorhydrique au composé de formule (I) est compris entre 5 et 50.

8. Procédé selon l'une des revendications précédentes, caractérisé en ce que l'acide fluorhydrique mis en œuvre est de l'acide fluorhydrique anhydre.

9. Procédé selon l'une des revendications précédentes, caractérisé en ce que les composés de formules (I) et (II) sont utilisés en quantité sensiblement équimolaires.

10. Procédé selon l'une des revendications précédentes, caractérisé en ce que on utilise une quantité de trifluorure de bore telle que la pression absolue de $BF_3$ dans l'enceinte réactionnelle soit comprise entre 6 et 20 bar.

11. Procédé selon l'une des revendications précédentes, caractérisé en ce que on opère à une température comprise entre −20 et 150° C.

## Patentansprüche

1. Verfahren zur Herstellung von Phenylketonen durch Reaktion eines Benzolderivats mit einer Carbonsäure, einem Vorläufer oder einem Derivat dieser Säure in Gegenwart von Bortrifluorid in einer solchen Menge, dass der absolute Druck des Bortrifluorids im Reaktionsgefäss über 1 bar liegt, und in Gegenwart von Fluorwasserstoffsäure als Lösungsmittel, dadurch gekennzeichnet, dass das Benzolderivat durch wenigstens eine α,α-Difluoralkoxy- oder wenigstens eine α,α-Difluorthioalkylgruppe desaktiviert wird.

2. Verfahren nach Anspruch 1, dadurch gekennzeichnet, dass das desaktivierte Benzolderivat die Formel hat:

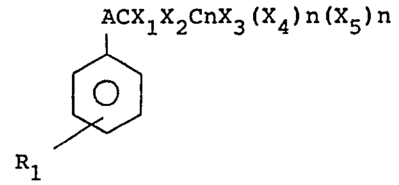

(I)

in der $X_1$ und $X_2$, die gleich oder verschieden sein können, Cl, Br, I oder F bedeuten, $X_3$, $X_4$ und $X_5$, gleich oder verschieden, H, Cl, Br, I oder F bedeuten, n 0 ist oder einen ganzzahligen Wert zwischen 1 und 5 annimmt, A O oder S bedeutet, und $R_1$ wenigstens ein Element bedeutet, das aus der Gruppe ausgewählt wird, die Wasserstoff und die Alkyl- und Alkoxyradikale mit 1 bis 6 Kohlenstoffatomen, und die mit wenigstens einer stärker desaktivierenden Gruppe als die Gruppierung $ACX_1X_2CnX_3(X_4)n(X_5)n$, OH, Cl, Br, I und F substituierten Phenyl- und Phenoxyradikale umfasst.

3. Verfahren nach Anspruch 2, dadurch gekennzeichnet, dass in der Formel (I) n = 0 oder 1 ist und $X_1$, $X_2$ und $X_3$ identisch sind.

4. Verfahren nach Anspruch 3, dadurch gekennzeichnet, dass $X_1$, $X_2$ und $X_3$ Fluor bedeuten.

5. Verfahren nach einem der vorangehenden Ansprüche, dadurch gekennzeichnet, dass die Carbonsäure, der Vorläufer oder das Derivat dieser Säure die Formel besitzt:

$$R_2-COX_6 \qquad (II)$$

in der $R_2$ einen aliphatischen oder aromatischen Rest, $X_6$ ein Halogen, eine von dem Anion einer anorganischen Säure abgeleitete Gruppe, OH, $OR_3$, $OCOR_4$, $NH_2$, $NHR_5$ oder $NR_6R_7$, wobei $R_3$, $R_4$, $R_5$, $R_6$ und $R_7$ ein aromatisches oder aliphatisches Radikal sind, bedeutet.

6. Verfahren nach Anspruch 5, dadurch gekennzeichnet, dass in der Formel (II) $R_2$ ein Alkyl-, Phenyl-, Alkylphenyl-, Phenylalkyl- oder Phenylradikal mit wenigstens einem Halogen-, $NO_2$-, CN-, $NH_2$- oder COOH-Substituenten bedeutet.

7. Verfahren nach einem der vorangehenden Ansprüche, dadurch gekennzeichnet, dass man eine derartige Menge Fluorwasserstoffsäure einsetzt, dass das Molverhältnis zwischen Fluorwasserstoffsäure und Verbindung der Formel (I) zwischen 5 und 50 liegt.

8. Verfahren nach einem der vorangehenden Ansprüche, dadurch gekennzeichnet, dass die eingesetzte Fluorwasserstoffsäure wasserfreie Fluorwasserstoffsäure ist.

9. Verfahren nach einem der vorangehenden Ansprüche, dadurch gekennzeichnet, dass die Verbindungen der Formeln (I) und (II) in ungefähr äquimolarer Menge eingesetzt werden.

10. Verfahren nach einem der vorangehenden Ansprüche, dadurch gekennzeichnet, dass man eine derartige Menge Bortrifluorid einsetzt, dass der absolute Druck des $BF_3$ in dem Reaktionsgefäss zwischen 6 und 20 bar liegt.

11. Verfahren nach einem der vorangehenden Ansprüche, dadurch gekennzeichnet, dass man bei einer Temperatur zwischen −20 und 150°C arbeitet.

## Claims

1. Process for preparing phenyl ketones by reaction of a benzene derivative with a carboxylic acid, a precursor or a derivative of this acid, in the presence of boron trifluoride in such quantity that the absolute pressure of boron trifluoride in the reaction enclosure is greater than 1 bar and in the presence of hydrofluoric acid as a solvent, characterized in that the benzene derivative is deactivated by means of at least one α,α-difluoroalkoxy group or by means of at least one α,α-difluorothioalkyl group.

2. Process according to Claim 1, characterized in that the deactivated benzene derivative has the formula:

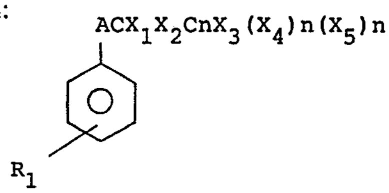

(I)

in which:

$X_1$ and $X_2$, which are identical or different, denote Cl, Br, I or F,

$X_3$, $X_4$ and $X_5$, which are identical or different, denote H, Cl, Br, I or F,

n is an integer greater than or equal to 0 and smaller than or equal to 5,

A denotes O or S, and

$R_1$ denotes at least one member chosen from the group comprising hydrogen and the alkyl and alkoxy radicals containing from 1 to approximately 6 carbon atoms, and the phenyl and phenoxy radicals substituted by at least one group which is more deactivating than the group $ACX_1X_2CnX_3(X_4)n(X_5)n$, OH, Cl, Br, I and F.

3. Process according to Claim 2, characterized in that, in the Formula (I), n = 0 or 1 and $X_1$, $X_2$ and $X_3$ are identical.

4. Process according to Claim 3, characterized in that $X_1$, $X_2$ and $X_3$ denote fluorine.

5. Process according to one of the principal claims, characterized in that the carboxylic acid, the precursor or the derivative of this acid corresponds to the formula:

$$R_2-COX_6 \qquad (II)$$

in which:

$R_2$ denotes an aliphatic or aromatic radical,

$X_6$ denotes a halogen, a group derived from the anion of an inorganic acid, OH, $OR_3$, $OCOR_4$, $NH_2$, $NHR_5$ or $NR_6R_7$, where $R_3$, $R_4$, $R_5$, $R_6$ and $R_7$ are an aromatic or aliphatic radical.

6. Process according to Claim 5, characterized in that, in the Formula (II), $R_2$ denotes an alkyl, phenyl, alkylphenyl, or phenylalkyl radical, or a phenyl radical containing at least one substituent halogen, $NO_2$, CN, $NH_2$ or COOH.

7. Process according to one of the preceding claims, characterized in that a quantity of hydrofluoric acid is employed such that the molar ratio of hydrofluoric acid to the compound of Formula (I) is between 5 and 50.

8. Process according to one of the preceding claims, characterized in that the hydrofluoric acid employed is anhydrous hydrofluoric acid.

9. Process according to one of the preceding claims, characterized in that the compounds of formulae (I) and (II) are employed in substantially equimolar quantities.

10. Process according to one of the preceding claims, characterized in that a quantity of boron trifluoride is employed such that the absolute pressure of $BF_3$ in the reaction enclosure is between 6 and 20 bar.

11. Process according to one of the preceding claims, characterized in that it is operated at a temperature of between −20 and 150°C.